**Europäisches Patentamt**

**European Patent Office**

**Office européen des brevets**

(11) Veröffentlichungsnummer: **0 337 352**
**A2**

## (12) EUROPÄISCHE PATENTANMELDUNG

(21) Anmeldenummer: 89106318.2

(51) Int. Cl.⁴: **A61M 1/28**

(22) Anmeldetag: 10.04.89

(30) Priorität: 15.04.88 DE 3812525

(43) Veröffentlichungstag der Anmeldung:
18.10.89 Patentblatt 89/42

(84) Benannte Vertragsstaaten:
**AT BE CH DE ES FR GB IT LI NL SE**

(71) Anmelder: **Fresenius AG**
**Gluckensteinweg 5**
**D-6380 Bad Homburg v.d.H.(DE)**

(72) Erfinder: **Bartz, Volker**
**Ludwig-Richter-Strasse 24**
**D-6300 Giessen(DE)**
Erfinder: **Steudle, Volker, Dr.**
**Rohnstrasse 26**
**D-6300 Giessen(DE)**

(74) Vertreter: **Görtz, Dr. Fuchs, Dr. Luderschmidt**
**Patentanwälte**
**Abraham-Lincoln-Strasse 7 Postfach 46 60**
**D-6200 Wiesbaden(DE)**

(54) **Dialysier- und Spüllösung zur intraperitonealen Verabfolgung.**

(57) Peritonealdialysierflüssigkeit mit üblicher Elektrolytzusammensetzung in physiologischen Mengen sowie mit Galaktose als osmotisch wirksamer Substanz in einer Menge von 1 - 70 g/l.

EP 0 337 352 A2

## Dialysier- und Spüllösung zur intraperitonealen Verabfolgung

Die Erfindung betrifft die Verwendung von Galaktose als osmotisch wirkende Substanz bei intraperitonealen Dialysier- und Spüllösungen.

Bei Patienten mit akuter oder chronischer Niereninsuffizienz muß die eingeschränkte Nierenfunktion durch alternative Verfahren kompensiert werden. Hierzu werden praktisch zwei Verfahren angewendet: Die Hämodialyse und die Peritonealdialyse. Bei der Hämodialyse wird das Blut der Patienten mit Hilfe einer künstlichen semipermeablen Membran extrakorporal gereinigt. Bei der Peritonealdialyse dient das Peritoneum als semipermeable Membran. Die Dialysierflüssigkeit wird über einen Katheter in die Peritonealhöhle eingebracht.

Aus der US-PS 4 308 255 wurde eine hypertonische Lösung bekannt, die als Dialysat für die Hämo- und Peritonealdialyse bei unter Verlust der Nierenfunktion leidenden Patienten anwendbar ist, und die die zur Auswaschung toxischer Substanzen bei der peritonealen Dialyse erforderliche Zeit verkürzen soll. Diese Lösung enthält 2 - 10 Gew.-% Dextran neben Natrium, Chlorid, Bicarbonat oder Laktat oder Acetat in bestimmten Mengenverhältnissen. Sie kann auch ggf. bis zu 5 Gew.-% Dextrose und bis zu 15 mg Zinkglukonat enthalten.

Eine zentrale Aufgabe einer Dialysierlösung für niereninsuffiziente Patienten besteht auf der einen Seite darin, überschüssiges Wasser und harnpflichtige Substanzen aufzunehmen, auf der anderen Seite in der Zufuhr von Substanzen, die metabolisch bedingt in zu geringer Konzentration im Organismus vorkommen (Elektrolytbilanz). Der Wasserentzug erfolgt bei der Peritonealdialyse durch osmotisch wirksame Substanzen, wie z.B. Glukose, Fruktose, Zuckeralkohole und -derivate oder andere unphysiologische Substanzen, wobei bis heute noch keine "optimale" osmotisch aktive Substanz gefunden wurde. Nach einer gewissen Equilibrierungszeit, d.h. nach konzentrationsabhängigem Austausch von Stoffen des Dialysats mit dem Blut, wird die "verbrauchte" Dialyselösung aus der Peritonealhöhle abgelassen und durch neue Lösung ersetzt. Weitere Anforderungen an eine Dialyselösung sind eine Zusammensetzung aus möglichst physiologischen, d.h. im gesunden Organismus natürlich vorkommenden Komponenten, um eine möglichst hohe Biokompatibilität zu erreichen und um weitere pathophysiologische Veränderungen, z.B. Akkumulation unphysiologischer oder nicht metabolisierbarer Substanzen, im Organismus zu vermeiden. Weitere Biokompatibilitätsparameter sind, daß die osmotisch wirksame Substanz keine systemische oder peritoneale Toxizität, keine Hemmung der lokalen Immunabwehrmechanismen, keine Immuntoxizität, eine nach Resorption schnelle Metabolisierung bei möglichst langsamer Resorptionsrate und physiologische pH- und Osmolaritätsbereiche aufweist.

Eine weitere Anforderung an eine Peritonealdialyselösung besteht darin, für den Patienten einen positiven nutritiven Effekt zu erzielen. Von praktischer Bedeutung sind Kohlenhydrate und Aminosäuren, insbesondere im Hinblick des täglichen Proteinverlustes der Patienten von 5 - 10 g in das Dialysat.

Weitaus am verbreitetsten ist die Verwendung von Glukose als osmotisch wirksame Substanz. Die Nachteile bestehen vor allem darin, daß Glukose von nahezu allen Mikroorganismen verstoffwechselt wird, wodurch die Gefahr einer Peritonitis mikrobieller Ursache steigt. Bei der Peritoneallavage von diffusen Peritonitiden mit glukosehaltigen Lösungen wächst das Risiko speziell von Candida-Infektionen überproportional. Des weiteren wird Glukose leicht aus dem Peritonealraum resorbiert. Die Blutglukosekonzentration ist erhöht (Hyperglykämie), mit der Folge einer erhöhten Insulinkonzentration (Hyperinsulinämie), die in Fettwechselstörungen (Hyperlipidämie) und Diabetes mellitus ihren Ausdruck finden kann. Speziell bei diabetischen Patienten ergeben sich von vornherein die aus der enteralen Ernährung her bekannten Probleme.

Aus der PCT-PA WO 83/00087 ist eine wässerige Lösung zur peritonealen Dialyse bekannt, die sich dadurch auszeichnet, daß die in herkömmlichen derartigen Lösungen vorhandenen monomeren Zucker, wie Glukose und Fruktose, oder dimeren Zucker, wie Saccharose oder Maltose, durch metabolisierbare Kohlehydratpolymere mit einem durchschnittlichen Polymerisationsgrad von zumindest 4 ersetzt sind; vorzugsweise ist das Kohlehydratpolymere ein Glokosepolymeres mit einem durchschnittlichen Polymerisationsgrad von 4 bis 10.

Der Abfall in der Osmolarität der Dialyselösung während des Verlaufs des peritonealen Dialyseverfahrens soll in Gegenwart derartiger Kohlehydratpolymeren geringer sein als bei herkömmlichen, Mono- oder Dissaccharide enthaltenen Dialyselösungen.

Bei Verwendung der wie Glukose als physiologisch zu kennzeichnenden Fruktose als osmotisch aktive Substanz werden zwar die Probleme der Hyperglykämie umgangen (Ernährung des Diabetikers), jedoch sind Irritationen des Peritoneums häufig.

Letzteres ist auch nicht bei Verwendung der aus der PCT-PA WO 82/03773 bekannten Dialyselösung

2

auszuschließen, welche eine wässerige Lösung physiologischer Salze in einer zur physiologischen Veträglichkeit mit Blut ausreichenden Konzentrationen sowie ein Gemisch physiologischer Aminosäuren mit Insulin in Anteilen umfaßt, die ausreichen, um die wesentlichen Assimilationen dieser Aminosäuren durch einen Patienten zu ermöglichen. Diese Lösung kann auch eine Quelle einer Kohlehydraternährung, wie z.B. Glukose, Glukosepolymere, Fruktose oder einen Zuckeralkohol enthalten; andere Kohlehydrate werden nicht genannt. Durch das Insulin soll erreicht werden, daß die Metabolisierung der Aminosäuren und gewünschtenfalls vorhandenen Glukose, die von der Dialyselösung in den Blutstrom des Patienten diffundieren, erleichtert wird.

Zudem führen erhöhte Plasmafruktosespiegel, insbesondere bei erhöhten Blutglukosespiegeln (Diabetes mellitus), über den Polyolweg zu einem Anstieg der Fruktosekonzentration im Linsengewebe des Auges. Da Fruktose im Gegensatz zu Glukose das Linsengewebe nicht mehr durch Permeation verlassen kann, kann der Anstieg der Fruktosekonzentration derartige Ausmaße annehmen, daß osmotisch wirksame Konzentrationen erreicht werden. Dies führt zu verstärktem Wassereinstrom in das Linsengewebe mit Schwellung und Störung der optischen Eigenschaften, die letztendlich zum Zustandsbild der Linsentrübung (diabetischer Katarakt) führen.

Nach Infusionen von Fruktoselösungen bei der parenteralen Ernährung können Laktatacidosen, Abfall der energiereichen Phosphate in der Leber, und ein Anstieg der Plasmaharnsäurespiegel beobachtet werden. Diese besonders für niereninsuffiziente Patienten dramatischen Stoffwechselstörungen werden dadurch verursacht, daß Fruktose in der Leber schneller als Glukose abgebaut wird. Die Phosphorylierung zu Fruktose-1-phosphat verursacht einen Abfall der Adenosintriphosphat- (ATP) und aktivierten Phosphatkonzentration ($P_i$), wobei der ATP-Abfall die 5-Nucleotidase, und der $P_i$-Abfall die Adenosinmonophosphat-(AMP)-Desaminase aktivieren. Demzufolge wird vermehrt Inosin zur Purinsynthese und Harnsäure gebildet. Eine Erhöhung des Harnsäurespiegels (Hyperuricämie) führt zu dem als Gicht bezeichneten, spezifischen Krankheitsbild, das sich durch Ablagerung von Urat in den Gelenken sowie anderen Geweben auszeichnet.

Andere osmotisch aktive Substanzen spielen derzeit bei Peritonealdialyselösungen eine untergeordnete Rolle, vor allem deswegen, weil aufgrund ihres unphysiologischen Charakters (mit Ausnahme von Aminosäuren und Peptiden) negative Langzeitwirkungen beobachtet werden, die hauptsächlich auf einer zu geringen oder zu langsamen Metabolisierung nach Resorption beruhen. Zu nennen sind hyperosmolare Plasmazustände bei Verwendung der Zuckeralkohole Sorbit und Xylit, Kumulation von Glukosepolymeren und Glycerin, allergische Reaktionen bei Gelatine und Toxizität von synthetischen Polymeren. Substanzen mit höherem Molekulargewicht (Hydroxyäthylstärke, Dextrane, Albumin) zeigen desweiteren eine geringere osmotische Wirksamkeit als kleinere Moleküle, so daß, um einen entsprechend hohen Wasserentzug zu erreichen (Ultrafiltration), sehr hohe Konzentrationen angewandt werden müssen, wodurch Schockreaktionen ausgelöst werden können (Dextranschock). Bei der Verwendung von Aminosäuren besteht die Gefahr der Induktion von Aminosäurenimbalanzen mit metabolischen Pathomechanismen, die sich langfristig negativ auf den Ernährungsstatus der Patienten auswirken können. Insbesondere bei Niereninsuffizienz muß eine optimale Aminosäurenkomposition mit anderen Substanzen noch gefunden werden. Darüber hinaus ergeben sich technische Probleme bei der Sterilisation von Aminosäuren oder Peptiden zusammen mit reduzierenden Zuckern als Kalorienträger durch Bildung toxischer Substanzen (Maillard-Reaktion).

Hinzu kommt ein Hauptproblem der peritonealen Dialyse, die Peritonitiskomplikation, die häufig zum Abbruch der Therapie führt: die genannten Substanzen dienen als hervorragende Substrate für relevante Mikroorganismen. Besonders bei Anwendung der Peritoneallavage bei postoperativen diffusen Peritonitiden ergeben sich durch gehäufte Pilzinfektionen (v.a. Candida albicans) enorme Schwierigkeiten wegen invasiven intraperitonealen Pilzwachstums.

Die der Erfindung zugrundeliegende Aufgabe ist es, osmotisch wirksame Dialyse- und Spüllösungen zur Verfügung zu stellen, die über längere Zeit an Patienten intraperitoneal verabfolgt werden können, ohne daß auf osmotische Erscheinungen zurückzuführende Komplikationen auftreten, wobei bei bislang bekannten Dialyse- und Spüllösungen auftretende mikrobielle und peritonealdialytische Komplikationen vermindert werden sollen und ein ausreichender Entzug von Wasser und harnpflichtigen Substanzen sowie eine Korrektur der Elektrolytbilanz gewährleistet sind. Desweiteren soll ein Beitrag zur Deckung des Kalorienbedarfs des Patienten sichergestellt werden.

Diese Aufgabe wird durch die erfindungsgemäße Dialyse-und Spüllösung zur intraperitonealen Verabfolgung gelöst, die durch eine wässerige Lösung mit einem Galaktosegehalt von 1 - 70 g/l als osmotisch wirksame Substanz gekennzeichnet ist.

Bei der Anwendung von erfindungsgemäßen galaktosehaltigen Dialyselösungen ergeben sich folgende Vorteile:
Galaktose ist biochemisch das der Glukose am ähnlichsten Monosaccharid. Die chemischen Eigenschaften, Summenformel und somit das Molekulargewicht sind identisch, ebenso die Resorptionsmechanismen. In der Leber wird Galaktose zu Glukose umgewandelt und kann dadurch leichter verstoffwechselt werden.

Das bedeutet ein verzögertes Auftreten von Glukose im Blut, was zur Verminderung von Glukosespitzen führt; dies ist ein beträchtlicher Vorteil im Falle insulinpflichtiger Patienten.

Die physiologischen Eigenschaften, technische Handhabung und - was besonders wichtig ist - auch die osmotische Aktivität entsprechen der der Glukose. Somit ergeben sich bei der Verwendung von Galaktose anstatt Glukose als osmotisch wirksame Substanz alle die diesbezüglich für Glukose bekannten positiven Eigenschaften ohne die Nachteile der Glukose. Die Ausbildung einer Hyperglykämie mit der Folge einer Hyperinsulinämie wird im wesentlichen vermindert, da die Umwandlung von Galaktose in Glukose in der Leber bedarfabhängig gesteuert wird. Exzessive Blutglukosekonzentrationen als Induktor für Diabetes mellitus und Hyperlipidämien werden vermieden.

Durch eine galaktosehaltige Peritonealdialysierflüssigkeit hervorgerufene erhöhte Plasmagalaktosespiegel sind physiologisch, im Gegensatz zu den meisten anderen bisher verwendeten osmotisch aktiven Peritonealdialyselösungen. Bei der enzymatischen Spaltung von Milchzucker (Laktose) -dem für den juvenilen Organismus zur Ernährung dienenden einzigen Kohlenhydrat - bestehend je aus einem Molekül der Monosaccharide Glukose und Galaktose, fällt nämlich Galaktose in großen Mengen physiologisch an.

Ein besonderer Vorteil, vor allem für tropische Länder, ist die Tatsache, daß Galaktose im Gegensatz zu Glukose kein Substrat für Candida albicans ist. Galaktose wird von Candida albicans nicht verstoffwechselt. Gefürchtete Pilzinfektionen werden infolgedessen vermieden.

Im Gegensatz zu Fruktose wird Galaktose nicht im Augenlinsengewebe eingelagert. Die Inosit- und Harnsäureproduktion wird nicht stimuliert.

Galaktose wird im Gegensatz zu Zuckeralkoholen, Glycerin, Di- und Oligosacchariden, Gelatine oder anderen höherwertigen Kohlehydraten parenteral resorbiert und vollständig metabolisiert. Es erfolgt keine Kumulation, und Galaktose entfaltet als osmotisch wirksame Substanz in der Peritonealdialyse keine Toxizität.

Die neue Dialyselösung umfaßt dieselben Komponenten wie bereits bekannte peritoneale Dialyselösungen, jedoch mit der Ausnahme, daß die osmotisch aktive Substanz, wie z.B. Glukose teilweise bis vollständig durch Galaktose ersetzt ist.

Die zur Anwendung kommenden Galaktosekonzentrationen sind abhängig von dem gemischten osmolaren Druck und liegen im Bereich von 1 - 70 g/l für Peritonealdialyselösungen. Vorteilhafterweise liegen sie im Bereich von 5 - 50, insbesondere 16- 25 g/l. Galaktose ist ein leicht erhältliches Handelsprodukt. Im Handel ist hochreine Galaktose mit einem Reinheitsgrad von mehr als 99% erhältlich. Die Gewinnung erfolgt technisch unter enzymatische oder saure Hydrolyse von Laktose.

Die Herstellung der neuen Dialyselösung kann nach den bekannten Verfahren zur Herstellung von glukosehaltigen Dialyselösungen erfolgen.

Wenn die erfindungsgemäße Dialyselösung neben der Galaktose auch Glukose als weitere fakultative, osmotisch wirksame Substanz enthält, so beträgt das Mengenverhältnis vorteilhafterweise 1:3 bis 3:1, vorzugsweise 1:1. Die Dialyselösung kann aber auch gewünschtenfalls nicht nur Glukose, sondern auch z.B. Fruktose, Sorbit, Xylit, Glycerin, modifizierte Gelatine, Kohlehydrate, Aminosäuren und/oder Peptide als weitere, fakultative osmotisch wirksame Substanz(en) enthalten.

Die Elektrolytsalze können in bekannter Weise in Form des Acetats, Laktats, Chlorids und/oder Bicarbonats vorliegen.

Die Ionenkonzentrationen in der erfindungegemäßen Dialyselösung betragen vorteilhafterweise 125-150, insbesondere 132-140 mmol/l $Na^+$; 0-8, insbesondere 0-4 mmol/l $K^+$; 0-3, insbesondere 0,5-2 mmol/l $Ca^{++}$; 0-2,5, insbesondere 0,3-1 mmol/l $Mg^{++}$; 10 - 60, insbesondere 30-50 mmol/l Ionen, ausgewählt aus der Gruppe Laktat-, Acetat- und Bicarbonationen, und Rest $Cl^-$.

Der osmotische Druck der erfindungsgemäßen Dialysierflüssigkeit beträgt vorteilhafterweise 300-700, insbesondere 320-550, vorzugsweise 350-450 mosm/l. Ein Beispiel für einen üblichen Zusatzstoff, der gewünschtenfalls in der Dialyselösung vorliegen kann, ist Insulin.

Beispiel 1:

In 1 Liter Wasser von Injektionsqualität wird eine Lösung von 16,5 g Galaktose und Elektrolytsalzen in Form des Laktats und Chlorids hergestellt.

4

| Na$^+$ | 132,0 | mmol/l |
|---|---|---|
| Ca$^{++}$ | 1,75 | mmol/l |
| Mg$^{++}$ | 0,75 | mmol/l |
| Cl$^-$ | 102,0 | mmol/l |
| Laktat$^-$ | 35,0 | mmol/l |
| Galaktosemonohydrat | 16,5 | g/l. |

Die Lösung wird filtriert und sterilisiert. Der theoretische osmotische Druck beträgt 355 mosm/l.

Beispiel 2:

Beispiel 1 wurde wiederholt, jedoch mit der Ausnahme, daß 46,75 g/l Galaktosemonohydrat eingesetzt wurden. Theoretischer osmotischer Druck: 507 mosm/l.

Beispiel 3:

Beispiel 1 wurde wiederholt, jedoch mit der Ausnahme, daß folgende Elektrolytsalze in folgenden Mengen eingesetzt wurden:

| Na$^+$ | 134,0 | mmol/l |
|---|---|---|
| K$^+$ | 2,0 | mmol/l |
| Mg$^{++}$ | 0,50 | mmol/l |
| Cl$^+$ | 105,5 | mmol/l. |
| (Nicht vollständig!) | | |

Theoretischer osmotischer Druck: 362 mosm/l.

**Ansprüche**

1. Wässerige Dialysier- und Spülflüssigkeit zur intraperitonealen Verabreichung, enthaltend Elektrolyte, wenigstens eine osmotisch wirkende Substanz und ggf. übliche Zusatzstoffe, gekennzeichnet durch einen Gehalt an Galaktose als osmotisch wirksame Substanz in einer Konzentration von 1 - 70 g/l.

2. Flüssigkeit nach Anspruch 1, gekennzeichnet durch einen osmotischen Druck im Bereich von 300 bis 700 mosm/l.

3. Flüssigkeit nach Anspruch 2, gekennzeichnet durch einen osmotischen Druck im Bereich von 320 bis 450 mosm/l.

4. Flüssigkeit nach Anspruch 3, gekennzeichnet durch einen osmostischen Druck im Bereich von 350 bis 450 mosm/l.

5. Flüssigkeit nach Anspruch 1, gekennzeichnet durch eine Galaktosekonzentration von 5 - 50 g/l.

6. Flüssigkeit nach Anspruch 5, gekennzeichnet durch eine Galaktosekonzentration von 16 - 25 g/l.

7. Flüssigkeit nach Anspruch 1, gekennzeichnet durch eine Konzentration von 125 - 150 mmol/l Na$^+$, 0 - 8 mmol/l K$^+$, 0 - 3 mmol/l Ca$^{++}$, 0 - 2,5 mmol/l Mg$^{++}$, 10 - 60 mmol/l Ionen ausgewählt aus der Gruppe : Laktationen, Acetationen, Bikarbonationen und Rest Cl$^-$.